Europäisches Patentamt

European Patent Office

Office européen des brevets

(19)

(11) **EP 1 541 169 A1**

(12) **EUROPEAN PATENT APPLICATION**

(43) Date of publication:
**15.06.2005  Bulletin 2005/24**

(51) Int Cl.⁷: **A61K 38/45**

(21) Application number: **03027839.4**

(22) Date of filing: **04.12.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **Aventis Pharma Deutschland GmbH 65929 Frankfurt am Main (DE)**

(72) Inventor: **The designation of the inventor has not yet been filed**

(54) **Use of limk-1, its analogues and ligands for the production of a medicament against a thrombus formation of blood clotting disease**

(57)    The present invention relates to LIMK-1, a LIMK-1-analogue or LIMK-1 ligand for the binding to GPIIb and/or activation or inhibition of GPIIb/IIIa downstream signaling, for the production of a medicament for the prevention or treatment of a thrombus formation or blood clotting disease, methods of screening method of screening a LIMK-1 analogue or a LIMK-1 ligand and a method for producing a medicament for the treatment of a thrombus formation or blood clotting disease.

EP 1 541 169 A1

**Description**

The present invention relates to LIMK-1, a LIMK-1-analogue or LIMK-1 ligand for the binding to GPIIb and/ or activation or inhibition of GPIIb/IIIa downstream signaling, for the production of a medicament for the prevention or treatment of a thrombus formation or blood clotting disease, methods of screening method of screening a LIMK-1 analogue or a LIMK-1 ligand and a method for producing a medicament for the treatment of a thrombus formation or blood clotting disease.

Platelets are the smallest blood cells, being only fragments of megakaryocyte cytoplasm, yet they have a critical role in normal haemostasis and are important contributors to thrombotic disorders. Platelet adhesion, activation and aggregation are a prerequisite reaction for the initiation of hemostasis, and play a role in the pathology of a wide variety of coronary, cerebral and peripheral vascular diseases (Bhatt et.al., 2003; George 2000; Moroi et.al., 1998; Rao et.al., 2000; Ruggeri 2002).

Platelet membranes contain high concentrations of integrins and other glycoproteins that are involved in platelet adhesion to extracellular matrix components (Lopez et.al., 1988; Phillips et.al., 1988; Shattil 1999). Collagen fibers and von Willebrand Factor (vWF) provide an important site for adhesion of platelets to the exposed subendothelium, trapping them at the site of vascular damage and enabling the formation of a monolayer of cells over the damaged area (Ruggeri 2002; Watson 1999). Collagen fibers and vWF, in concert with signals acting through G-protein coupled receptors (GPCRs) also stimulate platelet activation, leading to "inside-out" regulation of the integrin glycoprotein GPIIb/ IIIa (also known as ☐IIb☐3), secretion from dense and α-granules, generation of thromboxanes, and expression of procoagulant activity (Parise 1999; Ruggeri 2002; Shattil 1999). Also, on activation platelets change from the normal disc shape to a compact sphere with long dendritic extensions that facilitate adhesion. All these events support the hemostatic process.

The membrane proteins GPIIb (CD41) and GPIIIa (CD61) form the most abundant heterodimeric complex on the surface of platelets that binds fibrinogen, von Willebrand factor (vWF) and fibronectin. GPIIb/IIIa is expressed on the surface of resting platelets in an inactive conformation, which has low affinity for soluble fibrinogen. Following platelet activation by a multitude of important agonists such as collagen, ADP or thrombin, GPIIb/IIIa undergoes a conformational change which increases its binding affinity for soluble fibrinogen, resulting in platelet aggregation (Parise 1999; Ruggeri 2002; Shattil 1999). Signalling by GPIIb/IIIa involves several regions of the cytoplasmic tails. Several signalling proteins bind to the cytoplasmic domains of the GPIIb/GPIIIa integrin, including endonexin, calcium and integrin binding protein (CIB), Shc and Grb2. In addition, the sequence KVGFFKR in the cytoplasmic tail of the α subunit is involved in signalling, and lipid modified peptides corresponding to this region fully activate platelets (Stephens et.al., 1998). However, the exact series of signalling events which lead from platelet activation to fibrinogen receptor exposure is not known.

Since the demonstration that aspirin is effective on platelets and in the primary prevention of myocardial infarction, the prophylactic use of aspirin for thrombotic disorders has increased enormously. Thus, for several decades, antiplatelet therapy centered on the thromboxane pathway and its inhibition by aspirin.

Considering its relevance, adhesion is expected to be an attractive target for the development of antithrombotic drugs. However, the first series of compounds designed to prevent platelet aggregation, the αIIbβ3 antagonists, have not been successful as oral, long-term treatments. It is assumed that this due to the fact that, as with natural ligands, αIIbβ3 antagonists (many of which are based on receptor-recognition sequences in fibrinogen) actually trigger 'outside-in' signaling (Cox et.al., 2000). Thus, it may not be possible to interfere directly with the interaction between the ligand and the receptor without activating platelets.

During recent years, several antiplatelet therapies have been introduced, and the benefits of antiplatelet therapies ranging from aspirin, ticlopidine, and clopidrogel in thromboembolic disorders are documented (Konstantopoulos et.al., 2001; Mousa et.al., 2002; Weksler 2000). However, although all these different therapies add additional benefits for the treatment of thromboembolic disorders, there are still many cases where the efficacy of even combined treatments with the different agents turn out not to be sufficient. Furthermore, in many cases treatment with these antiplatelet therapies is associated with non-desirable side effects. Thus, novel approaches for the treatment of thromboembolic disorders are urgently needed.

Accordingly, an object of the present invention was to provide novel approaches for improved prevention and treatment of thrombus formation or blood clotting diseases and methods for the identification of novel targets for e.g. antithrombotic therapies.

Surprisingly it was now found that the LIMK-1 protein binds to GPIIb/IIIa. LIMK-1 is a 72.6 kDA protein (human: 647 amino acids) and a member of the LIM motif-containing protein kinase family. Its gene maps on-chromosome 7, at 7q11.23. The protein encompasses two N-terminal cysteine-rich LIM/double zinc finger motifs, a proline serine rich region with several putative casein kinase and MAP kinase recognition sites, a PDZ domain (PSD95/disc large/ZO-1) and a C terminal serine/threonine kinase domain. The zinc-finger like LIM-domains are postulated to mediate protein-protein interactions and have been described in nuclear and cytoskeletal proteins. It has been shown that the C-terminal kinase fragment of LIMK-1 binds to the LIM domain, and the LIM fragment dose-dependently inhibits the kinase catalytic activity of the kinase core fragment of LIMK-1. Thus, the N-terminal LIM domain negatively regulates the kinase activity of LIMK-1 by direct interaction with the C-terminal kinase domain (Nagata et.al., 1999). LIM-domain-kinase 1 (LIMK-1) regulates actin cytoskeletal reorganization via cofilin phosphorylation. Active cofilin leads to the depolymerization of actin. Phosphorylation of cofilin through LIMK-1 in consequence leads to the inactivation of the actin-depolymerizing activity of cofilin (Bierne et.al., 2001; Gohla et.al., 2002; Yang et.al., 1998). However, so far there has been no report about a potential role for LIMK-1 (or LIMK-2) in platelets.

Since LIMK-1 associates with the platelet integrin GPIIb, LIMK-1 triggers the shape changes of platelets induced by and necessary for platelet activation. Inhibition of LIMK-1 thus leads to the inhibition of platelet activation upon pro-thrombotic stimuli.

Therefore, inhibitors of LIMK-1 interfere with platelet activation and aggregation and therefore thrombus formation.

Accordingly, in a first aspect the invention is directed to the in vitro and in vivo use of LIMK-1 or a LIMK-1-analogue for the binding to GPIIb, in particular in order to modulate, preferably inhibit, the signal transduction downstream of the integrin GPIIb/IIIa.

LIMK-1 according to the present invention is any naturally occurring LIMK-1 protein. This includes LIMK-1 proteins and variants thereof of different species, preferably vertebrates, more preferably mammals, as well as splice variants. A preferred LIMK-1 protein is the human LIMK-1 protein. The amino acid sequence of the human LIMK-1 protein is disclosed in Mizuno et al., Oncogene 9: 1605-1612, 1994.

The term "LIMK-1 analogue" according to the present invention refers to a protein derived from LIMK-1 which binds GPIIb but is not LIMK-1. More particular it refers to an amino acid sequence which differs from the naturally occurring LIMK-1 sequence (i.e. wild-type polypeptide) in one or more amino acids. Such an analogue differs from the wild-type polypeptide in the substitution, insertion or deletion of one or more amino acids. Preferred are semi-conservative, more preferred conservative amino acid substitutions, whereby a residue is substituted by another with like characteristics. Typical substitutions are among the aliphatic amino acids, among the amino acids having aliphatic hydroxyl side chain, among the amino acids having acidic residues, among the amide derivatives, among the amino acids with basic residues, or the amino acids having aromatic residues. Typical semi-conservative and conservative substitutions are:

| Amino acid | Conservative substitution | Semi-conservative substitution |
|---|---|---|
| A | G; S; T | N; V; C |
| C | A; V; L | M; I; F; G |
| D | E; N; Q | A; S; T; K; R; H |
| E | D; Q; N | A; S; T; K; R; H |
| F | W; Y; L; M; H | I; V; A |
| G | A | S; N; T; D; E; N; Q |
| H | Y; F; K ;R | L; M; A |

| Amino acid | Conservative substitution | Semi-conservative substitution |
|---|---|---|
| I | V; L; M; A | F; Y; W; G |
| K | R; H | D; E; N; Q; S; T; A |
| L | M; I; V; A | F; Y; W; H; C |
| M | L; I; V; A | F; Y; W; C; |
| N | Q | D; E; S; T; A; G; K; R |
| P | V; I | L; A; M; W; Y; S; T; C; F |
| Q | N | D; E; A; S; T; L; M; K; R |
| R | K; H | N; Q; S; T; D; E; A |
| S | A; T; G ;N | D; E; R; K |

(continued)

| Amino acid | Conservative substitution | Semi-conservative substitution |
|---|---|---|
| T | A; S; G; N; V | D; E; R; K; I |
| V | A; L; I | M; T; C; N |
| W | F; Y; H | L; M; I; V; C |
| Y | F; W; H | L; M; I; V; C |

Changing from A, F, H, I, L, M, P, V, W or Y to C is semi-conservative if the new cysteine remains as a free thiol. Furthermore, the skilled person will appreciate that glycines at sterically demanding positions should not be substituted and that P should not be introduced into parts of the protein which have an alpha-helical or a beta-sheet structure.

The variant polypeptide differs in primary structure (amino acid sequence), but may or may not differ significantly in secondary or tertiary structure or in function relative to the wild-type. In any case the analogue shows an identity (homology) to the wild-type LIMK-1 of at least 75 %, preferably at least 80 %, more preferably at least 90 %, even more preferably at least 95 %, and most preferably at least 99 %.

The analogue can also be a portion of the LIMK-1 sufficient for binding to GPIIb. The portion comprises at least 30 amino acids, preferably at least 100 amino acids, more preferably at least 300 amino acids, even more preferably at least 450 amino acids, and most preferably at least 600 amino acids. This portion of the analogue can differ from the wild-type polypeptide portion in the substitution, insertion or deletion of one or more amino acids as detailed above. In one embodiment LIMK-1 or the LIMK-1 analogue can be fused to another molecule, e.g. a protein and/or a marker (e.g. as detailed above).

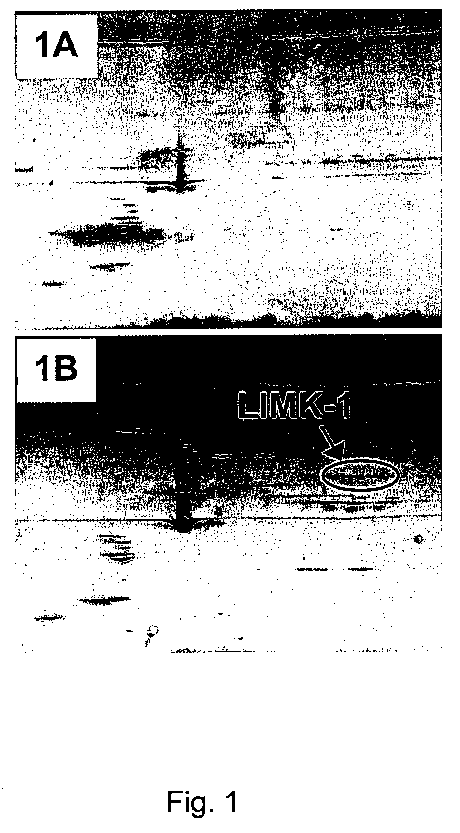

The term "binding to GPIIb" or "binds to GPIIb" refers to the specific binding of the prevailing compound (LIMK-1 or LIMK-1 analogue) to the GPIIb protein. A specific binding to the GPIIb protein according to the present invention includes, without limitation, binding with a dissociation constant $K_D$ of not exceeding $10^{-4}$ mol/l, preferably not exceeding $10^{-5}$ mol/l, more preferably not exceeding $10^{-6}$ mol/l. The dissociation constant $K_D$ can e.g, be determined using immunoprecipitation as set forth in the examples (see e.g. Fig. 1) by using varying the concentration of the tested compound, i.e. LIMK-1 or the LIMK-1 analogue, and a constant concentration of GPIIb. The concentration of the tested compound bound to GPIIb is determined by using e.g. a specific antibody against LIMK-1 or the LIMK-1 analogue. $K_D$ is determined according to the following equation:

$$B[L] = [L] / ([L] + K_D),$$

wherein [L] represents the concentration of the compound. $K_D$ is the dissociation constant of the tested compound and B[L] is the binding (%) at a particular concentration of the tested compound.

For detection the antibody may be labelled, which may suitably be a fluorophore, a chromophore, a radiolabel, a metal colloid, an enzyme, or a chemiluminescent or bioluminescent molecule. Suitable fluorophores and chromophores are disclosed in R. P. Haugland, Molecular Probes, Handbook of Fluorescent Probes and Research Chemicals, 5th Ed., Molecular Probes, Inc:, Eugene, Oreg., 1992. Examples of preferred fluorophores include fluorescein, rodamine, and sulfoindocyanine dye Cy5 (Mujumdar et al., Bioconjuga Chem. 4: 105, 1992). Preferred radiolabels include $^3$H, $^{14}$C, $^{32}$P, $^{33}$P, $^{35}$S, $^{99m}$Tc or $^{125}$I. Preferred enzymes include horseradish peroxidase, alkaline phosphatase, glucose oxidase, and urease.

GPIIb according to the present invention is any naturally occurring GPIIb protein. This includes GPIIb proteins and variants thereof of different species, preferably vertebrates, more preferably mammals, as well as splice variants. A preferred GPIIb protein is the human GPIIb protein, the amino acid sequence of which is disclosed in Poncz et al., J. Biol. Chem. 262: 8476-8482, 1987.

In one preferred embodiment of the invention the LIMK-1 analogue is an activator or inhibitor of the GPIIb/IIIa downstream signalling, preferably an inhibitor thereof.

Activators of the GPIIb/IIIa downstream signalling and activate the respective signal transduction pathway which leads to a detectable signal. Inhibitors block the GPIIb/IIIa downstream signaling at least partially. Activation and inactivation is a defined below. Activators and inactivators are identified as described below.

In another preferred embodiment of the invention LIMK-1 or the LIMK-1 analogue is used for the activation or inhibition of GPIIb/IIIa downstream signalling, more preferably for the inhibition thereof.

As detailed above the activation of GPIIb/IIIa downstream signalling leads to the activation of platelets orchestrated by inter alia a conformational change in the appearance of platelets from the normal disc shape to a compact sphere with long dendritic extensions. An activation of GPIIb/IIIa downstream signalling according to the present invention corresponds to an activation of at least 10 %, preferably at least 20 %, more preferably of at least 50 % and most preferably at least 80 % of the platelets when stimulated with LIMK-1 or the agonistic LIMK-1 analogue. The inhibition of GPIIb/IIIa downstream signaling leads consequently to an inhibition of platelet activation. An inhibiton of GPIIb/IIIa downstream signaling according to the present invention corresponds to an inhibition of at least 10 %, preferably at least 20 %, more preferably of at least 50 % and most preferably at least 80 % of platelets, when inhibited with or the inhibitory LIMK-1 analogue optionally in the presence of an GPIIb/GPIIIa activator.

Another subject of the invention is the use of a LIMK-1 ligand for the activation or inhibition of the GPIIb/IIIa downstream signalling, preferably for the inhibition thereof.

A LIMK-1 ligand is any compound molecule specifically binding to LIMK-1. A specific binding to the LIMK-1 according to the present invention includes, without limitation, binding with a dissociation constant $K_D$ of not exceeding $10^{-4}$ mol/l, preferably not exceeding $10^{-5}$ mol/l. The dissociation constant $K_D$ can determined as detailed with GPIIb or by using LIMK-1 protein and a suitable labelled LIMK-1 ligand. Suitable markers are detailed above. Furthermore, binding of LIMK-1 agonistic or antagonistic LIMK-1 ligands can be detected by activating or inactivating the kinase function of LIMK-1, respectively. Kinase functions can be easily detected by methods known to the one of skill in the art. Some of them are detailed below. For detection of antagonistic LIMK-1 ligands it may be necessary to activate LIMK-1 with e.g. PAK (p21-activated kinase) or ROCK (Rho kinase). Alternatively, binding of LIMK-1 ligands can be detected by measuring a more downstream signal in the signal transduction pathway, e.g. the activation or inactivation of platelets or the induction of their conformational change. A specific binding to the LIMK-1 according to the present invention includes, without limitation, binding with $EC_{50}$- and $IC_{50}$-values of each not exceeding $10^{-4}$ mol/l, preferably not exceeding $10^{-5}$ mol/l for agonistic and antagonistic LIMK-1 ligands, respectively. The determination and calculation of $EC_{50}$- and $IC_{50}$-values is known to the one skilled in the art.

A LIMK-1 may be a non-polymeric organic compound, a lipid, a carbohydrate, a peptide, preferably peptides with about 10 to about 300 amino acids, in particular with 10 to 50 amino acids. Especially preferred are small chemical molecules, in particular non-polymeric organic compounds, either synthesized in a laboratory or found in nature, with a preferred molecular weight of about 200 g/mole to about 1500 g/mole, in particular 400 g/mole to 1000 g/mole.

Alternatively, the LIMK-1 ligand of the present invention can be in the form of a natural product extract, either in crude or-in purified form. The extract can be produced according to standard procedures, such as water and/or alcohol and/or organic solvent extraction and/or column chromatography and/or precipitation from an animal, plant, fungi or microbial source, like snake poison, leaves or microbial fermentation broths.

In a preferred embodiment the LIMK-1 ligand is a LIMK-1 agonist or a LIMK-1 antagonist, more preferably a LIMK-1 antagonist.

Agonists bind to LIMK-1, induce changes, e.g. a conformational change, of the same and activate the respective signal transduction, e.g. activate the Kinase function of the LIMK-1, which leads to a detectable signal. Antagonists or blockers also bind to LIMK-1 but do in general not induce signal transduction. In the presence of an agonist, antagonists inhibit the agonist-induced signal transduction dose-dependently.

Another subject of the invention is the use of LIMK-1, a LIMK-1 analogue or a LIMK-1 ligand according to the invention for the production of a medicament for the prevention or treatment of a thrombus formation or blood clotting disease. A thrombus formation or blood clotting disease is a disease involving altered thrombus formation or blood clotting. As compared to a healthy human being the (disposition of) thrombus formation or blood clotting can be increased or reduced.

For the production of the medicament the LIMK-1, the LIMK-1 analogue or LIMK-1 ligand or its pharmaceutically acceptable salt has to be in a pharmaceutical dosage form in general consisting of a mixture of ingredients such as pharmaceutically acceptable carriers or auxiliary substances combined to provide desirable characteristics.

The formulation comprises at least one suitable pharmaceutically acceptable carrier or auxiliary substance. Examples of such substances are demineralised water, isotonic saline, Ringer's solution, buffers, organic or inorganic acids and bases as well as their salts, sodium chloride, sodium hydrogencarbonate, sodium citrate or dicalcium phosphate, glycols, such a propylene glycol, esters such as ethyl oleate and ethyl laurate, sugars such as glucose, sucrose and lactose, starches such as corn starch and potato starch, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils such as groundnut oil, cottonseed oil, corn oil, soybean oil, caster oil, synthetic fatty acid esters such as ethyl oleate, isopropyl myristate, polymeric adjuvans such as gelatin, dextran, cellulose and its derivatives, albumins, organic solvents, complexing agents such as citrates and urea, stabilizers, such as protease or nuclease inhibitors, preferably aprotinin, ε-aminocaproic acid or pepstatin A, preservatives such as benzyl alcohol, oxidation inhibitors such as sodium sulphite, waxes and stabilizers such as EDTA. Colouring agents, releasing agents, coating agents, sweetening, flavouring and perfuming agents, preservatives and antioxidants can also be present in the composition. The physiological buffer solution preferably has a pH of approx. 6.0-8.0, especially a pH of approx. 6.8-7.8, in particular a pH of approx. 7.4, and/or an osmolarity of approx. 200-400 milliosmol/liter, preferably of approx.

290-310 milliosmol/liter. The pH of the medicament is in general adjusted using a suitable organic or inorganic buffer, such as, for example, preferably using a phosphate buffer, tris buffer (tris(hydroxymethyl)aminomethane), HEPES buffer ([4-(2-hydroxyethyl)piperazino]ethanesulphonic acid) or MOPS buffer (3-morpholino-1-propanesulphonic acid). The choice of the respective buffer in general depends on the desired buffer molarity. Phosphate buffer is suitable, for example, for injection and infusion solutions. Methods for formulating a medicaments as well as suitable pharmaceutically acceptable carrier or auxiliary substance are well known to the one of skill in the art. Pharmaceutically acceptable carriers and auxiliary substances are a. o. chosen according to the prevailing dosage form and identified compound.

The pharmaceutical composition can be manufactured for oral, nasal, rectal, parenteral, vaginal, topic or vaginal administration. Parental administration includes subcutaneous, intracutaneous, intramuscular, intravenous or intraperitoneal administration.

The medicament can be formulated as various dosage forms including solid dosage forms for oral administration such as capsules, tablets, pills, powders and granules, liquid dosage forms for oral administration such as pharmaceutically acceptable emulsions, microemulsions, solutions, suspensions, syrups and elixirs, injectable preparations, for example, sterile injectable aqueous or oleaginous suspensions, compositions for rectal or vaginal administration, preferably suppositories, and dosage forms for topical or transdermal administration such as ointments, pastes, creams, lotions, gels, powders, solutions, sprays, inhalants or patches.

The specific therapeutically effective dose level for any particular patient will depend upon a variety of factors including the activity of the identified compound, the dosage form, the age, body weight and sex of the patient, the duration of the treatment and like factors well known in the medical arts.

The total daily dose of the compounds of this invention administered to a human or other mammal in single or in divided doses can be in amounts, for example, from about 0.01 to about 50 mg/kg body weight or more preferably from about 0.1 to about 25 mg/kg body weight. Single dose compositions may contain such amounts or submultiples thereof to make up the daily dose. In general, treatment regimens according to the present invention comprise administration to a patient in need of such treatment from about 10 mg to about 1000 mg of the compound(s) of the compounds of the present invention per day in single or multiple doses.

In a preferred embodiment the LIMK-1 analogue is an inhibitor of the GPIIb/IIIa downstream signalling. In another preferred embodiment the LIMK-1 ligand is a LIMK-1 antagonist.

Most of the patients with a thrombus formation or blood clotting disease suffer from diseases with increased (disposition of) thrombus formation or blood clotting. Therefore, they are in particular need for a medicament inhibiting thrombus formation or blood clotting. Consequently, drugs inhibiting thrombus formation or blood clotting such as inhibitory LIMK-1 analogues and antagonistic LIMK-1 ligands are preferred for the use for the production of a medicament.

In still another preferred embodiment the thrombus formation or blood clotting disease is a disease associated with increased thrombus formation or blood clotting.

Diseases with increased thrombus formation or blood clotting are known to the one skilled in the art and include e.g. arthero-thrombotic diseases.

In a more preferred embodiment the disease is an arthero-thrombotic disease, even more preferred a disease selected from the group consisting of myocardial infarction, unstable angina, acute coronary syndromes, coronary artery disease, reocclusion following coronary thrombolysis, occlusion during thromboplasty and coronary restenosis, stroke, transient ischemic attacks, pulmonary embolism, left ventricular dysfunction, secondary prevention of clinical vascular complications in patients with cardiovascular and cerebrovascular disease, atherosclerosis, comedication to vascular interventional strategies.

Yet, another embodiment of the invention is a method of screening a LIMK-1 analogue, wherein the method comprises the steps of:

    (a) providing a GPIIb protein and optionally at least one element of its downstream signalling;
    (b) providing a test compound; and
    (c) detecting the binding of the test compound to the GPIIb protein,

    wherein the test compound is derived from LIMK-1.

In general GPIIb and optionally at least one element of its downstream signalling are provided e.g. in an assay system and brought directly or indirectly into contact with a test compound derived from LIMK-1. The expression "test compound derived from LIMK-1" refers to a LIMK-1 analogue as defined above. Then, the binding of the test compound to the LIMK-1 protein is detected, wherein the binding can be detected by measuring the interaction between GPIIb and the test compound or by measuring the effect thereof on the downstream signalling.

The term "element of the downstream signalling of GPIIb/GPIIIa or GPIIb" refers to each molecule or ion being part of the signal transduction downstream of GPIIb/GPIIIa or GPIIb. It can be any element of any step in the signalling cascade. Preferably the element itself is a measurable signal or its produces a measurable signal. The element can be e.g. a second messenger or an enzyme. The signal can be e.g. a change in concentration of a substance or a conformational change.

Binding can be detected directly, i.e. by detecting the built complex, or indirectly, i.e. the effect of the building of the complex, which can be for example a downstream signal in the signal transduction pathway. Thereafter, suitable ligands can be analysed and/or isolated. Methods of measuring the binding of LIMK-1 analogues to GPIIb directly are detailed above.

In another embodiment of the invention the method of screening a LIMK-1 analogue according to invention comprises the following step instead of step (c):

(c') detecting the activation or inhibition of the GPIIb/IIIa and/or its downstream signaling.

Suitable functional assays for detecting activation or inhibition of LIMK-1 may e.g. involve the downstream signal transduction of GPIIb/GPIIIa. Activation or inhibition can e.g. be detected as detailed above by analyzing a downstream signal, e.g. the activation of platelets or their conformational change. It might be necessary to detect inhibition of GPIIb/GPIIIa signal transduction in the presence of a GPIIb and/or LIMK-1 stimulator, the signal of which is than inhibited by the inhibitory LIMK-1 analogue.

Still another embodiment of the invention is a method of screening a LIMK-1 ligand, wherein the method comprises the steps of:

(a) providing a LIMK-1 protein;
(b) providing a test compound; and
(c) detecting the binding of the test compound to the LIMK-1 protein.

In general LIMK-1 is provided e.g. in an assay system and brought directly or indirectly into contact with a test compound, in particular a biochemical or chemical test compound, e.g. in the form of a chemical compound library. Then, the binding of the test compound to the LIMK-1 protein is detected. Binding can be detected directly, i.e. by detecting the built complex, or indirectly, i.e. the effect of the building of the complex, which can be for example a downstream signal in the signal transduction pathway. Methods of measuring the binding of LIMK-1 ligand to LIMK-1 directly are detailed above. Thereafter, suitable ligands can be analyzed and/or isolated.

In another embodiment of the invention the method of screening a LIMK-1 ligand according to invention comprises the following step instead of step (c):

(c') detecting the activation or inhibition of the LIMK-1 or the GPII/IIIa downstream signaling, preferably the inactivation thereof.

Suitable functional assays for detecting activation or inactivation of LIMK-1 may e.g. involve the kinase function of LIMK-1. Methods of detecting kinase activity are known to the artisan (see below). In general these methods involve the transfer of a phosphate group, e.g. a labeled phosphate group such as a $^{32}$P or $^{33}$P labeled phosphate group, to an acceptor. Activation of LIMK-1 is therefore accompanied with increased phosphate transfer and inactivation with a diminished phosphate transfer. It might be necessary to detect inactivation of LIMK-1 in the presence of a LIMK-1 stimulator, the signal of which is than inhibited by the antagonistic LIMK-1 ligand. Activation or inactivation can also be detected by measuring a downstream signal, e.g. the activation of platelets or their conformational change.

Another subject of the present invention is a method of screening a test compound interacting with GPIIb/IIIa and/or its downstream signaling, wherein the method comprises the steps of:

(a) providing a LIMK-1 protein;
(b) providing GPIIb and/or at least one element of its downstream signaling;
(c) providing a test compound; and
(d) detecting the effect of the compound to the GPIIb/GPIIIa downstream signaling.

In general LIMK-1 and GPIIb and/or at least one element of its downstream signal transduction are provided e.g. in an assay system and brought directly or indirectly into contact with a test compound, in particular a biochemical or chemical test compound, e.g. in the form of a chemical compound library. Then, the effect of the test compound to the GPIIb/GPIIIa signal transduction is detected as described above. Thereafter, suitable ligands can be analyzed and/or isolated.

In one preferred embodiment whole cells, e. g. platelets, are used for the screening methods of the present invention thereby providing GPIIb/GPIIIa and further elements of its downstream signaling. In this case the detected signal can be the change of shape of the platelets.

The test compound interacts with GPIIb and/or its downstream signaling, when the binding LIMK-1 or analogue thereof to GPIIb in the presence of the test compound differs from the one in the absence of the test compound. The signal transduction can be either increased or reduced, preferably reduced. The detected difference in downstream signaling amounts least 10 %, preferably at least 20 %, more preferably of at least 50 % and most preferably at least 80 % and is calculated as ratio of the signals in the presence and in the absence of the test compound.

In another embodiment of the screening methods of the invention the test compound is provided in the form of a chemical compound library. Chemical compound libraries include are plurality of chemical compounds and have been assembled from any of multiple sources, including chemically synthesized molecules and natural products, or have been generated by combinatorial chemistry techniques. They are especially suitable for high throughput screening. They may be comprised of chemical compounds of a particular structure or compounds of a particular creature such as a plant. In the context with the present invention the chemical compound library is preferably a library comprising proteins and polypeptides or small molecules.

In still another embodiment of screening method for screening a LIMK-1 analogue or LIMK-1 ligand according to the invention the binding of the test compound to said GPIIb protein or said LIMK-1 protein or its effect on the GPIIb and/or its downstream signaling is detected in a heterogeneous or homogeneous assay. As used herein, a heterogeneous assay is an assay which includes one or more washing steps, whereas in a homogeneous assay such washing steps are not necessary. The reagents and compounds are only mixed and measured.

In a preferred embodiment the heterogeneous assay is an ELISA (enzyme linked immuno sorbent assay), a DELFIA (dissociation enhanced lanthanide fluoro immuno assay), an SPA (scintillation proximity assay) or a flashplate assay.

ELISA (enzyme linked immuno sorbent assay)-based assays are offered by various companies. The assays employ random peptides that can be phosphorylated by a kinase, such as LIMK-1. Kinase-containing samples are usually diluted into a reaction buffer containing e.g. ATP and requisite cations and then added to plate wells. Reactions are stopped by simply removing the mixtures. Thereafter, the plates are washed. The reaction is initiated e.g. by the addition of a biotinylated substrate to the kinase. After the reaction, a specific antibody is added. The samples are usually transferred to pre-blocked protein-G plates and after washing e. g streptavidin-HRP is added. Thereafter, unbound streptavidin-HRP (horseradish peroxidase) is removed, the peroxidase colour reaction is initiated by addition of the peroxidase substrate and the optical density is measured in a suitable densitometer.

DELFIA (dissociation enhanced lanthanide fluoro immuno assay)-based assays are solid phase assay. The antibody is usually labelled with Europium or another lanthanide and the Europium fluorescence is detected after having washed away unbound Europium-labelled antibodies.

SPA (scintillation proximity assay) and the flashplate assay usually exploit biotin/avidin interactions for capturing radiolabelled substrates. Generally the reaction mixture includes the kinase, a biotinylated peptide substrate and $\gamma$-[P$^{33}$]ATP. After the reaction, the biotinylated peptides are captured by streptavidin. In the SPA detection, streptavidin is bound on scintillant containing beads whereas in the flashplate detection, streptavidin is bound to the interior of the well of scintillant containing microplates. Once immobilized, the radiolabelled substrate is close enough to the scintillant to stimulate the emission of light.

In another preferred embodiment the homogeneous assay is a TR-FRET (time-resolved fluorescence resonance energy transfer) assay, a FP (fluorescence polarization) assay, an ALPHA (amplified luminescent proximity homogenous assay), an EFC (enzyme fragment complementation) assay or a gene assay.

TR-FRET (time-resolved fluorescence resonance energy transfer)-based assays are assays which usually exploit the fluorescence resonance energy transfer between Europium and APC, a modified allophycocyanin or other dyes with overlapping spectra such as Cy3/Cy5 or Cy5/Cy7 (Schobel, U. et al. (1999) Bioconjugate Chem. 10, 1107-1114). After excitation e.g. of Europium with light at 337 nm, the molecule fluoresces at 620 nm. But if this fluorophore is close enough to APC, the Europium will transfer its excitation energy to APC, which fluoresces at 665 nm. The kinase substrate is usually a biotin-labeled substrate. After the kinase reaction, Europium-labeled-(P)-specific antibodies are added along with streptavidin-APC. The phosphorylated peptides bring the Europium-labeled antibody and the streptavidin-APC into close contact. The close proximity of the APC to the Europium fluorophore will cause a quenching of the Europium fluorescence at benefit of the APC fluorescence (FRET).

Fluorescence polarization (FP)-based assays are assays which use polarized light to excite fluorescent substrate peptides in solution. These fluorescent peptides are free in solution and tumble, causing the emitted light to become depolarized. When the substrate peptide binds to a larger molecule, however, such as (P)-Tyr, its tumbling rates are greatly decreased, and the emitted light remains highly polarized. For a kinase assay there are generally two options:

(a) A fluorescent phosphopeptide tracer is bound to a (P)-specific antibody. Phosphorylated products will compete the fluorescent phosphopeptide from the antibody resulting in a change of the polarization from high to low.
(b) A phosphorylated substrate peptide binds to the phosphospecific antibody resulting in a change of polarization from low to high.

ALPHA (amplified luminescent proximity homogenous)-based assays, are assays which rely on the transfer of singlet oxygen between donor and acceptor beads brought into proximity by a phosphorylated peptide. Upon excitation at 680 nm, photosensitisers in donor beads convert ambient oxygen to singlet-state oxygen, which diffuses up to a distance of 200 nm. Chemiluminescent groups in the acceptor beads transfer energy to fluorescent acceptors within the bead, which then emits light at approximately 600 nm.

EFC (enzyme fragment complementation)-based assays or equivalent assays can be used in particular for high-throughput screening of compounds. The EFC assay is based on an engineered β-galactosidase-enzyme that consists of two fragments - the enzyme acceptor (EA) and the enzyme donor (ED). When the fragments are separated, there is no β-galactosidase activity, but when the fragments are together they associate (complement) to form active enzyme. The EFC assay utilizes an ED-analyte conjugate in which the analyte may be recognized by a specific binding protein, such as an antibody or receptor. In the absence of the specific binding protein, the ED-analyte conjugate is capable of complementing EA to form active β-galactosidase, producing a positive luminescent signal. If the ED-analyte conjugate is bound by a specific binding protein, complementation with EA is prevented, and there is no signal. If free analyte is provided (in a sample), it will compete with the ED-analyte conjugate for binding to the specific binding protein. Free analyte will release ED-analyte conjugate for complementation with EA, producing a signal dependent upon the amount of free analyte present in the sample.

Yet, in another embodiment the screening method for screening a LIMK-1 analogue or LIMK-1 ligand according to the invention is carried out on an array by binding either GPIIb or LIMK-1 to the array. Methods for preparing such arrays using solid phase chemistry and photolabile protecting groups are disclosed, for example, in US 5,744,305. These arrays can also be brought into contact with test compound or compound libraries and tested for interaction, for example binding or changing conformation.

In still another embodiment the screening method for screening a LIMK-1 analogue or LIMK-1 ligand according to the invention is carried out using whole cells. Preferably, platelets are used for such test, since they can be easily received from blood donors. Alternatively, cell lines, optionally transfected cell lines, can be used. Such cell lines include but are not limited to megacaryocyte cell lines (e.g. DAMI or MEG-1), HEK 293 cells (primary human embryonal kidney), 3T3 cells (murine embryonal fibroblasts), CHO cells (Chinese hamster ovary), COS-7 cells (African green monkey cell line), HeLa cells (human epithelioid cervical carcinoma), JURKAT cells (human T-cell leukaemia), BHK 21 cell (hamster normal kidney, fibroblast),_ and MCF-7 cells (human breast cancer). Using whole cells is advantageous in contrary to membranes, since intracellular substrates, enzymes etc. need not to be added to the test system. Furthermore, whole cells in multiwell plates are particularly suitable for high trough put screening test and automated test systems.

In another embodiment the screening method for screening a LIMK-1 analogue or LIMK-1 ligand according to the invention is carried out in a robotics system. Advantageously the method of the present invention is carried out in a robotics system e.g. including robotic plating and a robotic liquid transfer system, e.g. using microfluidics, i.e. channeled structured.

In still another embodiment the screening method for screening a LIMK-1 analogue or LIMK-1 ligand according to the invention is carried out in form of a high-through put screening system. In such a system advantageously the screening method is automated and miniaturized; in particular it uses miniaturized wells and microfluidics controlled by a roboter.

Another subject of the invention is a method for producing a medicament for the prophylaxis or treatment of a thrombus formation or blood clotting disease, wherein the method comprises the steps of:

(a) carrying out a method of screening according to the present invention,
(b) providing suitable amounts of a detected test compound, and
(c) formulating the detected test compound with one or more pharmaceutically acceptable carriers or auxiliary substances as detailed above.

A thrombus formation or blood clotting disease is a disease involving altered thrombus formation or blood clotting. As compared to a healthy human being the (disposition of) thrombus formation or blood clotting can be increased or reduced. Diseases with increased thrombus formation or blood clotting are known to the one skilled in the art and include e.g. arthero-thrombotic diseases.

In a preferred embodiment the disease is an arthero-thrombotic disease.

In a still more preferred embodiment of the invention the arthero-thrombotic disease is selected from the group consisting of myocardial infarction, unstable angina, acute coronary syndromes, coronary artery disease, reocclusion following coronary thrombolysis, occlusion during thromboplasty and coronary restenosis, stroke, transient ischemic attacks, pulmonary embolism, left ventricular dysfunction, secondary prevention of clinical vascular complications in patients with cardiovascular and cerebrovascular disease, atherosclerosis, comedication to vascular interventional strategies.

The following Figures and Examples shall explain the present invention in more detail without limiting the scope of the invention.

FIGURES

Figure 1 shows the detection of LIMK1 with 2D gel electrophoresis of GPIIb immunoprecipitates. Immunoprecipitates of human resting platelet extracts (3.9 mg) with control IgG (Fig.1A) or specific antibodies against GPIIb (Fig.1 B) were separated by IEF (isoelectric focusing) in a first step on a pH 3-10 gradient. Subsequently, proteins were separated in a second dimension on 10 % SDS -PAGE. Gels were colloidal coomassie-stained and analyzed. Protein spots visible on the gel with specific GPIIb antibodies only were excised, digested with trypsin (in-gel digest), extracted and analyzed by MALDI-TOF MS.

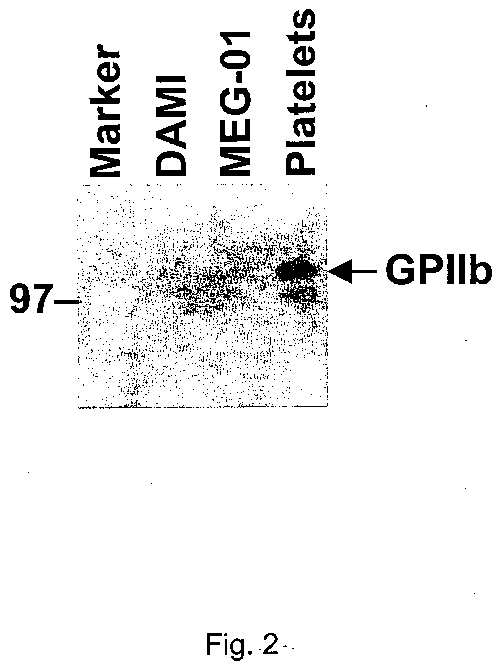

Figure 2 shows the detection of the integrin GPIIb following immunoprecipitation with antibodies against LIMK-1. Immunoprecipitates of human platelet extracts (2 mg), as well as the human megakaryocyte cell lines DAMI and MEG-01 with a specific antibody against LIMK-1 were separated by 4-12% SDS-PAGE. Proteins were transferred to nitrocellulose membrane by electrotransfer. The integrin GPIIb was detected using a specific antibody.

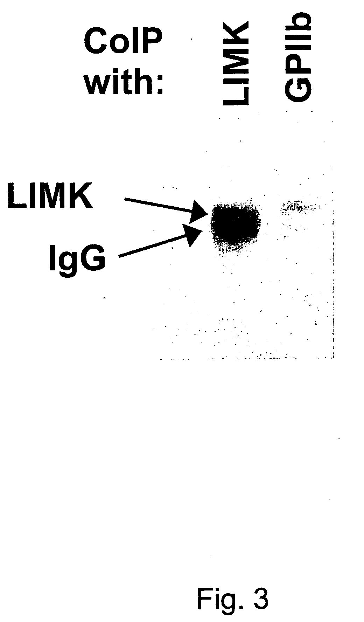

Figure 3 shows the detection of LIMK-1 following immunoprecipitation with antibodies against LIMK-1 and GPIIb. Immunoprecipitates of human platelet extracts with the specific antibodies against LIMK-1 and GPIIb were separated by 10 % SDS-PAGE. Proteins were transferred to nitrocellulose membrane by electrotransfer. LIMK-1 was detected using the specific antibody.

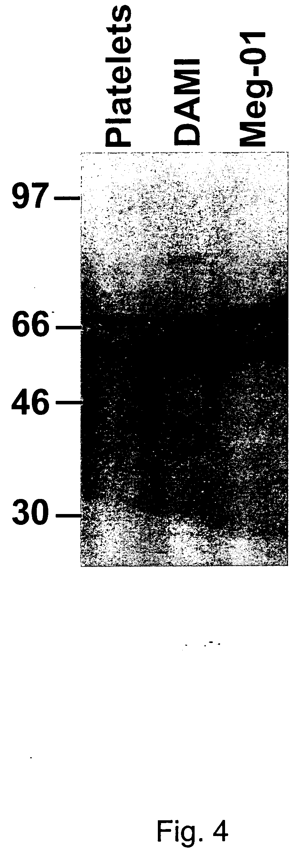

Figure 4 shows the detection of LIMK-1 in extracts of platelets as well as DAMI and Meg-01 cells. Equal protein amounts from extracts of human platelets (50μg) were separated by 4-12% SDS-PAGE. Proteins were transferred to nitrocellulose membrane by electrotransfer. LIMK-1 was detected by using a specific antibody.

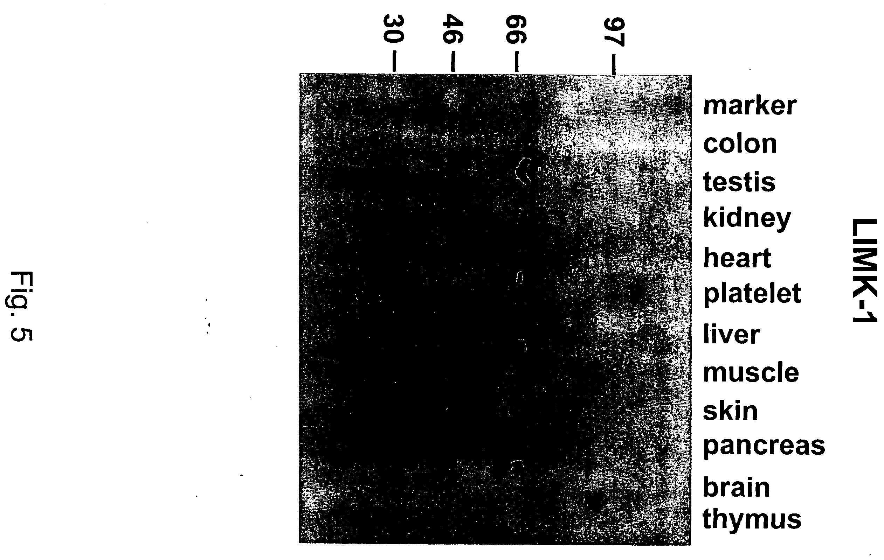

Figure 5 shows the detection of LIMK-1 in extracts of diverse human tissues. Equal protein amounts from extracts of human colon, testis, kidney, heart, platelet, liver, muscle, skin, pancreas, brain, and thymus tissue were separated by 4-12% SDS-PAGE. Proteins were transferred to nitrocellulose membrane by electrotransfer. LIMK-1 was detected by using a specific antibody.

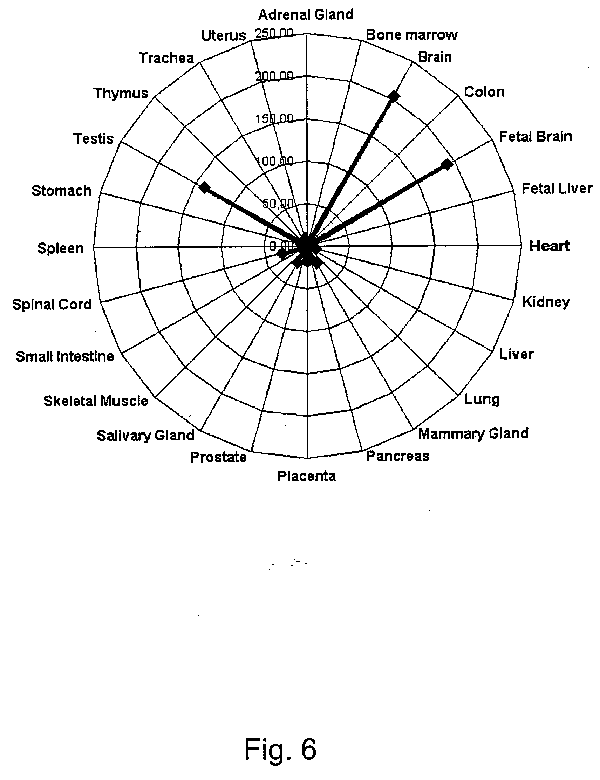

Figure 6 shows the detection of LIMK-1 transcripts by quantitative Taqman analysis in diverse human tissues. mRNA from human bone marrow, colon, testis, kidney, heart, platelet, liver, fetal liver, lung, mammary gland, placenta, prostate, salivary gland, intestine, spinal cord, spleen, stomach, trachea, uterus, muscle, pancreas, brain, fetal brain, and thymus tissue were analyzed by Taqman analysis.

EXAMPLES

1. MATERIAL & METHODS

1.1 Preparation of platelets (thrombocytes)

Thrombocytes were prepared out of thrombocyte concentrate (of 4 donors) from the blood bank. All steps were carried out at room temperature. Thrombocyte concentrate was diluted with Tyrode's buffer (pH 7.4; 137 mmol/l NaCl, 2.7 mmol/l KCl, 12 mmol/l NaHCO$_3$, 0.36 mmol/l NaH$_2$PO$_4$, 1 mmol/l MgCl$_2$, 10 mmol/l HEPES, 5.6 mmol/l Dextrose and the resulting suspension was centrifuged at 120 x g for 15min without brake. Then prostaglandin E$_1$ (PGE$_1$ 0.5μg/ml) was added to the supernatant, the mixture was incubated for 5 min at room temperature and centrifuged at 650 x g for 15min without brake. The supernatant was discarded, the pellet resuspended in Tyrode' buffer (with 0.1 %BSA) and PGE$_1$ (0.5 μg/ml) added. The mixture was incubated for 5 min at room temperature and subsequently centrifuged at 650 x g for 5min without brake. The supernatant was discarded, the pellet resuspended in Tyrode's buffer (without BSA), PGE$_1$ (0.25μg/ml) added the mixture incubated (5min at room temp.) and centrifuged (650 x g, 15min without brake). Again the supernatant was discarded, the pellet resuspended in Tyrode's buffer (without BSA) and the cells were frozen in liquid nitrogen and then stored at-80°C until use.

1.2 Cell culture

Cultures of human DAMI and Meg-01 megakaryocyte cell lines were grown in Dulbecco's modified Eagles's medium (DMEM) containing 10% fetal calf serum (FCS) and penicillin/streptomycin (37° C, 5% $CO_2$). Cells were washed, harvested, and cell pellets were stored at -80°C before preparation of extracts.

1.3 Treatment of platelets for immunoprecipitation

Frozen platelet-pellets were thawed in 1-2 ml of lysis buffer (see below) and then resuspended by pipetting up and down, followed by sonication (10 pulses, 30 %). Pre-fractionation was performed for the enrichment of soluble proteins in the supernatant by centrifuging the platelets (5 min, 3.000 x g).

Lysis and wash buffers used for Figure 1:
Lysis buffer (pH 8): 10 mmol/l Tris, 140 mmol/I NaCl, 1% Triton, 0.05% SDS, 1 tab. Complete, 200 µmol/l pefabloc (4-(2-Aminoethyl)-benzen sulfonyl fluoride hydrochloride))
Wash buffer (pH 8): 10 mmol/I Tris, 140 mmol/I NaCl, 1% Triton
Lysis and wash buffers used for Figure 2:
Lysis buffer (pH 7.5): 50 mmol/I Tris, 50 mmol/l NaCl, 0.5% Triton, 0.05% SDS, 1 mmol/I $Na_3VO_4$, 1 tab. Complete, 1 µg/ml Pepstatin, 5 mmol/I NaF
Wash buffer (pH 7.5): 50 mmol/I Tris, 50 mmol/I NaCl, 0.1% Triton
Lysis and wash buffers used for Figure 3:
Lysis buffer (pH 7.5): 50 mmol/I Tris, 150 mmol/I NaCl, 1% Triton, 1 mmol/I $Na_3VO_4$, 1 tab. Complete,1 µg/ml Pepstatin, 50 mmol/I NaF
Wash buffer (pH 7.5): 50 mmol/I Tris, 150 mmol/I NaCl, 1% Triton

1.4 Immunoprecipitation

Protein-A-Sepharose (Amersham Biosciences, Uppsala, Sweden) as well as Protein-G-Agarose (Roche Diagnostics GmbH, Mannheim, Germany) was used for immunoprecipitation depending on the type of antibody. All steps were carried out at 4°C.
Beads were washed with wash buffer (see above) 3 times prior to use. Platelet lysates were precleared by 1 h incubation (Fig.1+2, not in 3) with the bead-material and subsequent centrifugation (3 min, 500 x g). The platelet lysates were incubated with the antibody for 1 h and then the beads were added for immonoprecipitation over night. After that, the beads were washed with wash buffer (see above) 3 times and then elution was performed by incubation with IEF-rehydration buffer (for 2 D-gel analysis) or lysis-buffer (for 1 D-gel analysis).

1.5 Antibody specifications

GPllb (Santa Cruz Biotechnology, Inc. in Santa Cruz, California., USA; Cat-No. sc-7310) for immunoprecipitation (Protein A)
GPIIb (Biotrend Chemikalien GmbH, Cologne, Germany; Cat-No. 6065) for Western Blot (1:1000)
LIMK (Santa Cruz Biotechnology, Inc. in Santa Cruz, California., USA; Cat-No. sc-5576) for immunoprecipitation (Protein G) and for Western Blot (1:250).

1.6 2D-Gel electrophoresis

1st Dimension gel electrophoresis was performed using Biorad Protean IEF Cell (IPG-strips: 11 cm, pH 3-10) (Biorad Laboratories, Hercules, CA, USA) and the following IEF-rehydration-buffer (8 mol/I Urea, 0.5 % CHAPS, 10 mmol/I DTT, 0.2 % Biolytes, 0.001% Bromophenol Blue). For active rehydration IPG-strips were left overnight at 50 V with 185 µl of sample volume. IEF (isoelectric focusing) was performed with linear ramp, final voltage of 8000 V and a total of at least 40000 Vhrs. Reduction/Alkylation prior to 2nd dimension was carried out for 10 min in each equilibration buffer (pH 8.8; 6 mol/I urea, 2% SDS, 0.375 mol/I Tris, 20% glycerol, 130 mmol/I DTT or 135 mmol/I iodoacetamide).

2nd Dimension gel electrophoresis was performed using Criterion 4-20% gels at 150 V and 25 mmol/I Tris, 192 mmol/I Glycine, 0.1% SDS as running buffer.

1.7 1D-Gel electrophoresis

1 D-gel electrophoresis was performed using the X-Cell Sure Lock System (Novex-system; Invitrogen GmbH, Karlsruhe, Germany) as well as MOPS-SDS as running buffer and a voltage of 150 V.

1.8 Electrotransfer/Western-Blot (Semidry)

For electrotransfer the Multiphor II System (discontinuous buffer system) (Amersham, Biosciencies, Uppsala, Sweden) was used. Anode buffer 1 was 0.3 mol/I Tris, 20% methanol, anode buffer 2 was 25 mmol/I Tris, 20% methanol and kathode buffer was 40 mmol/I aminocaproic acid, 0.01 % SDS, 20% methanol.
Proteins were blotted onto nitrocellulose-sheets (Schleicher&Schuell BioScience GmbH, Dassel/Relliehausen, Germany; Protran BA85 0.45um) using 0.8 mA/cm$^2$.

1.9 Staining techniques

The gels were stained with colloidal coomassie (Neuhoff et al., 1988) or silver (modified after Blum et al., 1987). For modified silver staining was the gel was fixed in 40 % ethanol/10 % acetic acid for 1 h, washed twice in 30 % ethanol, once in water for each 20 min, sensitized in 0.02% $Na_2S_2O_3$ for 1 min, washed in water (3 x 20 sec), incubated in cold 0.1 % $AgNO_3$ at 4°C for 20min, washed in water (3 x 20 sec and than 1 x 1 min), developed in 3% $NaCO_3$/0.05 % formalin and washed once more in water for 20 sec. Finally staining was terminated in 5 % acetic acid.

1.10 Imaging

Antibodies were detected by chemiluminescence (ECL plus, Amersham Biosciences, Uppsala, Sweden; Cat-No. RPN2132) after 5 min exposure to Hyperfilm ECL (Amersham Biosciences, Uppsala, Sweden; Cat-No. RPN2132) and developed by Adefo-Developer (00009) and Adefo-Fixator (00062) (ADEFO-CHEMIE GmbH, Nürnberg, Germany).

1.11 In-gel digest

In-gel digest was carried out according to Pandey et al., 2000. Briefly, gel-spots were excised manually and Trypsin (porcine; Promega GmbH, Mannheim, Germany; Cat-No. V511A) was used for cleavage.

1.12 MALDI-TOF mass-spectrometry

The MALDI-TOF mass spectroscopy was carried out using a Voyager DE-STR MALDI-TOF workstation (Applied Biosystems, Foster City, CA, USA). Samples were prepared by the dried-droplet method and spotted onto a 2 x 96 well teflon-coated sample plate, using □-cyano-hydroxy-cinnamic acid (3 mg/ml in acetonitrile/trifluoroacetic acid) as a matrix. The detection range run from 700-4000 Da.

1.13 Protein determination

The protein content of the samples was determined using BCA-Kit (Pierce Chemical Company, Rockford, Illinois, USA) according to the manufacturer's instructions (detection at 562nm).

1.14 Tissue distribution patterns

50 μg of protein per lane was applied on the gels. Different human tissues were purchased from BioCat GmbH (Heidelberg, Germany). Detection of LIMK out of platelets and megakaryocytes was enhanced by previous enrichment in the supernatant (centrifugation 15 000 x g, 15 min or 3 000 x g, 5 min for Fig. 4 and Fig. 5, respectively).

1.15 Taqman analysis

For the amplification the following PCR protocol was used. All of the following reagents for the amplification were from Applied Biosystems (Foster City, USA): 20 ng of genomic DNA; 1 unit of TaqGold polymerase; 1 x Taq polymerase buffer; 500 μM of dNTP; 2,5 mmol/I of $MgCl_2$; 200 nmol/I of each amplification primer pair; $H_2O$ ad 5 μl.

Amplification program for PCR:

95°C x 10min x 1 cycle

95°C x 30sec
70°C x 30sec x 2 cycles;

95°C x 30sec
65°C x 30sec x 2 cycles;

95°C x 30sec
60°C x 30sec x 2 cycles;

95°C x 30sec
56°C x 30sec
72°C x 30sec x 40 cycles;

72°C x 10min
4°C x 30sec x 1 cycle;

1.16 Equipment

Experiments were carried out using the Biacore 3000 highest performance research system (Biacore International SA, 79111 Freiburg, Germany) equipped with CM5-chips and Ni-NTA-chips.

2. RESULTS

2.1 LIMK-1 interacts with the integrin GPIIb

Using co-immunoprecipitation with commercially available specific antibodies to GPIIb, we enriched protein complexes with GPIIb. These complexes were separated by 2-dimensional gel electrophoresis (Fig. 1). The result obtained in this experiment clearly demonstrates that LIMK-1 co-precipitates with the integrin GPIIb in human platelet extracts.

In order to verify these results, we have used immunoprecipitations of platelet extracts as well as extracts from the human megakaryocyte cell lines DAMI and MEG-01 with the commercially available, specific antibody to LIMK-1 for subsequent detection of GPIIb by Western analysis (Fig. 2). These results confirmed the finding that the integrin GPIIb is associated with LIMK-1 in human platelets. The interaction between GPIIb and LIMK-1 could not be shown in the human megakaryocyte cell lines DAMI and MEG-01. These megakaryocyte cell lines are the precursor cell lines that after differentiation produce platelets (Fugman et.al., 1990; George 2000; Greenberg et.al., 1988; Ogura et.al., 1988; Schick et.al., 1998; Takeuchi et.al., 1998; Vittet et.al., 1992). However, for our experiments we used these cell lines in the non-differentiated form. When analyzing the expression levels of the GPIIb integrin in these non-differentiated megakaryocyte cell lines we found that expression of GPIIb is much lower in these cell lines compared to expression in platelets. This explains why we did not detect the interaction between the integrin and LIMK-1 in these cell lines.

To further verify our results, we used immunoprecipitations of platelet extracts with the commercially available, specific antibodies to LIMK-1 and GPIIb for subsequent detection of LIMK-1 by Western analysis (Fig. 3). Again these results confirmed our previous finding that the integrin GPIIb is associated with LIMK-1 in human platelets.

2.2 Tissue distribution for LIMK-1

Using the specific antibody for LIMK-1, we were analyzing the expression of LIMK-1 in platelets as well as in the megakaryocyte cell lines DAMI and MEG-01 (Fig. 4). Thus, using the specific antibody for LIMK-1, we confirmed the expression of LIMK-1 in platelets as well as in the megakaryocyte cell lines DAMI and MEG-01.

Furthermore, in order to analyze the expression of LIMK-1 in different human tissues, we separated human tissue extracts by gel electrophoresis for subsequent Western analysis (Fig. 5). These results show that LIMK-1 is expressed in testis, heart, platelet, muscle, and brain tissue. Although the expression is not absolutely specific to platelets, LIMK-1 is not expressed ubiquitously in all tissues. This finding is confirmed by Taqman analysis (Fig. 6), showing expression of LIMK-1 in brain, fetal brain, and testis. Platelets are not represented in this Taqman panel.

**Claims**

1. A use of LIMK-1 or a LIMK-1-analogue for the binding to GPIIb.

2. The use according to claim 1, wherein the LIMK-1 analogue is an activator or inhibitor of the GPIIb/IIIa downstream signaling, preferably an inhibitor thereof.

3. The use according to claim 1 or 2 for the activation or inhibition of the GPIIb/IIIa downstream signaling , preferably for the inhibition thereof.

4. A use of a LIMK-1 ligand for the activation or inhibition of the GPIIb/IIIa downstream signaling , preferably for the inhibition thereof.

5. The use according to claim 4, wherein the LIMK-1 ligand is a LIMK-1 agonist or a LIMK-1 antagonist, preferably a LIMK-1 antagonist.

6. The use of LIMK-1, a LIMK-1 analogue or a LIMK-1 ligand for the production of a medicament for the prevention or treatment of a thrombus formation or blood clotting disease.

7. The use according to claim 6, wherein the LIMK-1 analogue is an inhibitor of the GPIIb/IIIa downstream signaling or wherein the LIMK-1 ligand is a LIMK-1 antagonist.

8. The use according to claim 6 or 7, wherein the disease is a disease associated with increased thrombus formation or blood clotting.

9. The use according to one of the claims 6 to 8, wherein the disease is an arthero-thrombotic disease.

10. The use according to one of the claims 6 to 9, wherein the disease is selected from the group consisting of myocardial infarction, unstable angina, acute coronary syndromes, coronary artery disease, reocclusion following coronary thrombolysis, occlusion during thromboplasty and coronary restenosis, stroke, transient ischemic attacks, pulmonary embolism, left ventricular dysfunction, secondary prevention of clinical vascular complications in patients with cardiovascular and cerebrovascular disease, atherosclerosis, comedication to vascular interventional strategies.

11. A method of screening an LIMK-1 analogue, wherein the method comprises the steps of:

    (a) providing a GPIIb protein and optionally at least one element of its downstream signaling;
    (b) providing a test compound; and
    (c) detecting the binding of the test compound to the GPIIb protein,

    wherein the test compound is derived from LIMK-1.

12. The method according to claim 11 comprising the following step instead of step (c):

    (c') detecting the activation or inhibition of the GPIIb/IIIa and/or its downstream signaling .

13. A method of screening a LIMK-1 ligand, wherein the method comprises the steps of:

    (a) providing a LIMK-1 protein;
    (b) providing a test compound; and
    (c) detecting the binding of the test compound to the LIMK-1 protein.

14. The method according to claim 13 further comprising the following step instead of step (c):

    (c') detecting the activation or inhibition of the LIMK-1 or the GPII/IIIa downstream signaling, preferably the inactivation thereof.

15. A method of screening a test compound interacting with GPIIb/IIIa and/or its downstream signaling cascade, wherein the method comprises the steps of:

(a) providing a LIMK-1 protein;
(b) providing GPIIb and/or at least one element of its downstream signaling;
(c) providing a test compound; and
(d) detecting the interaction of the compound to the LIMK-1 protein.

16. The method according to any of the claims 13 to 15, wherein the test compound is provided in the form of a chemical compound library.

17. The method according to any of the claims 11 to 16, wherein the binding of the test compound to said GPIIb protein or said LIMK-1 protein or its effect on the GPIIb and/or its downstream signaling is detected in a heterogeneous or homogeneous assay.

18. The method according to claim 17, wherein the heterogeneous assay is an ELISA (enzyme linked immune sorbent assay), a DELFIA (dissociation enhanced lanthanide fluoro immune assay) or a SPA (scintillation proximity assay).

19. The method according to claim 17, wherein the homogeneous assay is a TR-FRET (time-resolved fluorescence resonance energy transfer) assay, a FP (fluorescence polarization) assay, an ALPHA (amplified luminescent proximity homogenous assay), an EFC (enzyme fragment complementation) assay.

20. The method according to any of the claims 11 to 19, wherein the method is carried out on an array.

21. The method according to any of the claims 11 to 17, wherein the method is carried out using whole cells.

22. The method according to any of the claims 11 to 21, wherein the method is carried out in a robotics system.

23. The method according to any of the claims 11 to 22, wherein the method is carried out using microfluidics.

24. The method according to any of the claims 11 to 23, wherein the method is a method of high-through put screening of a LIMK-1 analogue or a LIMK-1 ligand.

25. A method for producing a medicament for the prophylaxis or treatment of a thrombus formation or blood clotting disease, wherein the method comprises the steps of:

(a) carrying out the method according to any of the claims 11 to 24,
(b) providing suitable amounts of the detected compound, and
(c) formulating the detected test compound with one or more pharmaceutically acceptable carriers or auxiliary substances.

26. The method of claim 25, wherein the disease is an arthero-thrombotic disease.

27. The method of claim 26, wherein the arthero-thrombotic disease is selected from the group consisting of myocardial infarction, unstable angina, acute coronary syndromes, coronary artery disease, reocclusion following coronary thrombolysis, occlusion during thromboplasty and coronary restenosis, stroke, transient ischemic attacks, pulmonary embolism, left ventricular dysfunction, secondary prevention of clinical vascular complications in patients with cardiovascular and cerebrovascular disease, atherosclerosis, comedication to vascular interventional strategies.

Fig. 1

Fig. 2

Fig. 3

Fig. 4

# LIMK-1

Fig. 5

Fig. 6

**European Patent Office**

**EUROPEAN SEARCH REPORT**

Application Number

EP 03 02 7839

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 403 353 B1 (YAN CHUNHUA ET AL) 11 June 2002 (2002-06-11) * column 2, line 26; claim 2 * --- | 1-10 | A61K38/45 |
| A | WO 01 04308 A (INCYTE GENOMICS INC ;AZIMZAI YALDA (US); YUE HENRY (US); TANG Y TO) 18 January 2001 (2001-01-18) * page 1, line 20-24 * ----- | 1-27 | |

| | TECHNICAL FIELDS SEARCHED (Int.Cl.7) |
|---|---|
| | A61K |

The present search report has been drawn up for all claims

| Place of search | Date of completion of the search | Examiner |
|---|---|---|
| MUNICH | 17 March 2004 | Ludwig, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 03 02 7839

This annex lists the patent family members relating to the patent documents cited in the above-mentioned European search report.
The members are as contained in the European Patent Office EDP file on
The European Patent Office is in no way liable for these particulars which are merely given for the purpose of information.

17-03-2004

| Patent document cited in search report | | Publication date | Patent family member(s) | | Publication date |
|---|---|---|---|---|---|
| US 6403353 | B1 | 11-06-2002 | US | 6340583 B1 | 22-01-2002 |
| | | | US | 2003166215 A1 | 04-09-2003 |
| | | | CA | 2442052 A1 | 03-10-2002 |
| | | | EP | 1379631 A2 | 14-01-2004 |
| | | | WO | 02077171 A2 | 03-10-2002 |
| WO 0104308 | A | 18-01-2001 | AU | 6091900 A | 30-01-2001 |
| | | | CA | 2378989 A1 | 18-01-2001 |
| | | | EP | 1196568 A1 | 17-04-2002 |
| | | | JP | 2003504060 T | 04-02-2003 |
| | | | WO | 0104308 A1 | 18-01-2001 |

EPO FORM P0459

For more details about this annex : see Official Journal of the European Patent Office, No. 12/82